# FASCICULE DE BREVET EUROPEEN

(11) **EP 0 656 890 B1**
(45) Date de publication et mention de la délivrance du brevet: **03.01.2001**
(21) Numéro de dépôt: 94920514.0
(22) Date de dépôt: 27.06.1994
(51) Int. Cl.: C07D 233/84

(54) **NOUVEAU PROCEDE DE PREPARATION DE L'ERGOTHIONEINE**
NEUES VERFAHREN ZUR HERSTELLUNG VON ERGOTHIONEIN
NOVEL METHOD FOR PREPARING ERGOTHIONEINE

(30) Priorité: 28.06.1993 FR 3007839; 22.12.1993 FR 9315457
(43) Date de publication de la demande: 14.06.1995
(73) Titulaire: OXIS Isle of Man, Limited, Douglas 1M1 3DB, Isle of Man (GB)
(72) Inventeur: YADAN, Jean-Claude, F-75011 Paris (FR); XU, Jinzhu, F-94200 Ivry-sur-Seine (FR)
(74) Mandataire: Portal, Gérard
(86) Numéro de dépôt international: FR9400769
(87) Numéro de publication internationale: WO9500494

(56) Documents cités:
- JOURNAL OF THE CHEMICAL SOCIETY, 1951, LETCHWORTH GB pages 2215 - 2217 H. HEATH ET AL. '2-Mercaptoglyoxalines. Part I. The Synthesis of Ergothioneine.' cité dans la demande

## Description

La présente invention a pour objet un procédé original de préparation des différentes formes optiques de l'ergothionéine, en particulier de la D,L-ergothionéine et de la L-(+)-ergothionéine, qui est une molécule naturelle.

### I/ Etat de l'art antérieur:

La L-ergothionéine ou 2-mercapto-N_{α},N_{α},N_{α} -triméthyl-L-histidine est une molécule naturelle qui a été isolée d'un champignon de l'ergot de seigle *Claviceps purpurea* (voir la communication de Tanret C., C.R. Acad., Sci., 149 (1909), pages 222-224). Elle a été ultérieurement identifiée dans les érythrocytes et le foie de rat, puis dans de nombreux autres tissus animaux et en particulier chez l'Homme (voir l'article de Melville D.B., dans Vitam. Horm., 17, (1958), pages 155-204). Elle est exclusivement biosynthétisée par les champignons et les mycobactéries. Chez les végétaux, l'assimilation de l'ergothionéine se fait par les racines après synthèse fongique à l'intérieur des conidies. Son apport chez l'Homme est uniquement d'origine alimentaire.

Bien que le rôle biologique exact de l'ergothionéine reste incertain, ses propriétés antioxydantes sont bien documentées (voir l'article de Hartman P.E. dans Meth. Enzymol., 186, (1990), pages 310-318 et l'article de Akanmu D. et Coll., dans la revue Arch. Biochem. Biophys., 288, (1991), pages 10-16). Ainsi, l'ergothionéine peut être utilisée en tant qu'additif alimentaire ou en cosmétique, ou même en médecine, grâce à ses propriétés antioxydantes.

L'ergothionéine a été obtenue soit par voie microbiologique (voir le document JP-A-43-020 716 (1968) de Miyoshi T. et Sakai H.), soit par voie chimique (voir l'article de Heath H. et Coll. dans la revue J. Chem Soc., pages 2215-17, (1951) et l'article de Sunko D.E. et Wolf G. dans la revue J. Chem. Soc., pages 4405-4406, (1958)). Cette dernière voie, pour être réalisable, implique de surmonter les trois difficultés suivantes :
* la β-élimination très facile, en milieu alcalin, du groupement triméthylammonium conduisant à l'acide 2-mercaptourocanique ;
* la méthylation d'un groupement azoté en présence d'un groupement soufré;
* la racémisation très aisée, en milieu faiblement alcalin, de l'ergothionéine en raison de sa structure de type bétaïne.

De plus, la préparation à l'échelle industrielle de ce composé implique un rendement global au moins supérieur ou égal à 20 %.

L'intermédiaire-clef des préparations synthétiques de l'art antérieur est la 2-mercaptohistidine, obtenue soit par dégradation du noyau imidazole de l'histidine puis synthèse du noyau 2-mercaptoimidazole à l'aide de thiocyanate de potassium (voir l'article de Heath précité) ; soit par synthèse totale (voir l'article précité de Sunko, ainsi que l'article de Hegedus B. dans la revue Helv. Chim. Acta, 38, (1955), pages 22-27)).

La préparation de l'ergothionéine, à partir de la 2-mercaptohistidine, utilise ensuite toujours le même procédé (voir l'article de Heath précité) : ce qui constitue le principal écueil de ces préparations. En effet, l'étape de méthylation finale nécessite l'utilisation de grandes quantités d'oxyde d'argent, d'hydrogène sulfuré pour éliminer les sels d'argent, puis d'acide phosphotungstique ; le tout dans des conditions particulièrement sévères. De plus, la mise en oeuvre de cette dernière étape est laborieuse et apparait inapplicable à une préparation à l'échelle industrielle de l'ergothionéine. Enfin, l'ergothionéine est obtenue partiellement voire totalement racémisée ; et le rendement global faible de ces préparations ne dépasse jamais 9 %.

Compte tenu de l'intérêt bien connu, résultant des documents précédents cités, que présente l'ergothionéine en général et plus particulièrement la L-(+)-ergothionéine, la Demanderesse a cherché une nouvelle synthèse de ces molécules qui permettrait d'éviter les inconvénients susmentionnés, tout en obtenant le produit désiré dans des conditions très douces avec un bon rendement global et avec une excellente pureté optique, de préférence supérieure à 98 %.

Ce but est atteint selon l'invention qui fournit une nouvelle synthèse de l'ergothionéine basée sur un procédé original de synthèse du noyau 2-mercapto-imidazole.

L'étape-clef du procédé selon l'invention est basée sur une stratégie originale selon laquelle on introduit l'atome de soufre sur le noyau imidazole par une réaction dont le mécanisme s'apparenterait à un mécanisme de type "Addition Nucléophile-Ouverture du Cycle-Refermeture du Cycle" ( ANORC).

Plus précisément, l'invention a pour objet un procédé de synthèse chimique des différentes formes optiques en particulier (D,L) et (L) de l'ergothionénine, caractérisé en ce qu'il comprend essentiellement les étapes consistant à :
a) préparer ou utiliser un sel d'ester d'histidine N_{α},N_{α} -diméthylée, si nécessaire optiquement actif;
b) traiter ce composé par un halogènothionoformiate d'alkyle, d'alkènyle ou d'aryle, en particulier de phényle, en présence d'une base;
c) après protection du substituant soufré du composé obtenu, transformer ce composé protégé en un composé de type triméthylammonium ; et
d) libérer l'ergothionéine désirée par saponification, ou hydrolyse acide, selon le cas.

L'ester d'histidine est avantageusement un ester d'alkyle en C₁-C₆, notamment un ester méthylique.

Le sel est avantageusement un halogènure, notamment un dichlorhydrate.

On peut partir d'un composé commercial tel que le dichlorhydrate de l'ester méthylique de la L-histidine que l'on méthyle.

On peut également partir d'un sel de l'ester méthylique de la N_{α},N_{α}-diméthyl-L-histidine obtenu selon le procédé de Reinhold V.N. et Coll. décrit dans la revue J. Med. Chem., 11, (1968), pages 258-260.

L'halogènothionoformiate d'aryle utilisé est avantageusement le chorothionoformiate de phényle.

Lors du traitement par ce dernier composé; la base utilisée est par exemple le bicarbonate de sodium ou une amine, ou alkylamine, en particulier la triéthylamine.

La protection du substituant soufré peut être effectuée par acylation de préférence au moyen d'un halogénoformiate usuel dans ce cas tel que par exemple un halogénoformiate d'éthyle ou de phényle, en particulier le chloroformiate.

La transformation en composé de type triméthylammonium est réalisée au moyen d'un agent alkylant comme par exemple un halogénure de méthyle, en particulier l'iodure de méthyle, ou le sulfate de diméthyle.

Ce dernier composé est saponifié par exemple au moyen d'une amine, en particulier une alkylamine, de préférence la triéthylamine, dans un mélange eau/alcool. Un alcool actuellement préféré est le méthanol.

Selon une variante, notamment dans le cas où l'on part d'un composé optiquement actif pour obtenir une ergothionéine également optiquement active, le composé de type triméthylammonium précédent est avantageusement hydrolysé au moyen d'une solution acide, comme par exemple une solution d'acide chlorhydrique concentrée, de préférence en présence d'un large excès d'un mercaptan. Comme mercaptan, on utilise avantageusement un alkyle ou aryle mercaptan, de préférence l'acide β-mercaptopropionique.

Selon un mode particulier de réalisation, l'invention a pour objet un procédé de synthèse chimique de la D,L-ergothionéine caractérisé en ce qu'il comprend essentiellement les étapes consistant à :
a) préparer ou utiliser un sel d'ester d'histidine N_{α},N_{α} -diméthylée ;
b) traiter ce composé par un halogènothionoformiate d'alkyle, d'alkènyle ou d'aryle, en particulier de phényle, en présence d'une base;
c) protéger le substituant soufré du composé obtenu, de préférence par acylation, encore de préférence au moyen d'un halogénoformiate d'alkyle ou de phényle, en présence d'une base, de préférence la triéthylamine ;
d) traiter le composé protégé obtenu par un agent méthylant, de préférence un halogénure de méthyle dans un alcool ; et
e) libérer la D,L-ergothionéine désirée par saponification de préférence au moyen d'une amine dans un mélange eau/alcool.

Selon un mode préféré de réalisation, l'invention a pour objet un procédé de synthèse chimique de la L-(+)-ergothionéine caractérisé en ce qu'il comprend essentiellement les étapes consistant à :
a) préparer ou utiliser un sel d'ester d'histidine N_{α},N_{α} -diméthylée ;
b) traiter ce composé par un halogènothionoformiate d'alkyle, d'alkènyle ou d'aryle, en particulier de phényle, en présence d'une base;
c) protéger le substituant soufré du composé obtenu, de préférence par acylation, encore de préférence au moyen d'un halogénoformiate d'alkyle ou de phényle, en présence d'une base, de préférence la triéthylamine ;
d) traiter le composé protégé obtenu par un agent méthylant, de préférence un halogénure de méthyle dans un alcool ; et
e) libérer la L-(+)-ergothionéine désirée par hydrolyse acide, de préférence au moyen d'une solution d'acide fort.

Selon un mode de réalisation avantageux, l'halogénure de méthyle précité est l'iodure de méthyle et l'alcool précité est le méthanol.

Selon un mode de réalisation préféré, l'hydrolyse acide pour libérer la L-(+)-ergothionéine est réalisée en présence d'un large excès d'un mercaptan. Comme mercaptan, on utilisera avantageusement un alkyle ou aryle mercaptan. Un mercaptan particulièrement préféré actuellement est l'acide β-mercaptopropionique. L'acide fort précité est avantageusement une solution d'acide chlorhydrique concentré.

Avantageusement, le mercaptan est présent dans un rapport molaire d'au moins 50 par rapport à l'ester, et encore mieux d'au moins 70. Il est à noter que le mercaptan fonctionne comme un piégeur de carbocation généré au cours de l'hydrolyse acide, afin d'éviter toute réaction parasite avec l'atome de soufre du noyau 2-mercapto-imidazole.

L'atmosphère réactionnelle n'est pas critique, mais pour des raisons pratiques les exemples suivants ont été réalisés en travaillant sous atmosphère inerte d'azote.

Dans l'un ou l'autre des aspects précédents de l'invention on peut réaliser le traitement du composé par un halogénothionoformiate d'alkyle, d'alkènyle ou d'aryle en présence d'une base dans un solvant polaire. Cette base peut être choisie parmi un bicarbonate, une amine ou une alkylamine, en particulier diéthylamine ou triéthylamine.

Selon un mode de réalisation préféré pour l'obtention d'un meilleur rendement, on réalise ce traitement en deux étapes. Une première étape consiste à réaliser la réaction dans une solution aqueuse d'une base faible de préférence à un pKa inférieur à environ 9, en particulier un bicarbonate, avantageusement en présence d'un solvant polaire favorisant la solubilité du chlorothionoformiate, par exemple de l'éther éthylique. Cette réaction se fait avantageusement par addition goutte à goutte de l'hagénothionoformiate dissous dans le solvant polaire à la température ambiante dans ladite solution contenant le composé à traiter dans la solution aqueuse de bicarbonate. L'intermédiaire comportant le cycle imidazole ouvert est récupéré dans la phase organique du solvant, par exemple l'éther éthylique, avantageusement séché par exemple sur MgSO₄ pour éliminer l'eau restante, et évaporé pour conduire à une huile. Cette huile est ensuite traitée par une autre base de préférence dont le pKa est supérieur à environ 10, encore de préférence organique, avantageusement type amine ou alkylamine, en particulier diéthylamine ou triéthylamine, à température ambiante pendant plusieurs heures ; avantageusement en présence d'un solvant polaire. De préférence, ce solvant polaire est un éther, en particulier le tétrahydrofuranne. Le solvant est ensuite éliminé pour obtenir le produit de la réaction recherché.

Il est à noter que la première étape est particulièrement délicate parce que le risque de racémisation du composé optiquement actif de départ est très grand. Il a pu être observé que ce risque de racémisation est particulièrement élevé en présence d'une base forte. De ce fait, la première étape doit être réalisée en présence d'une base faible et le bicarbonate est particulièrement adapté pour cette réaction. D'autre part, le rendement global de formation du cycle imidazole est particulièrement élevé, c'est-à-dire supérieur à 70 % en comparaison d'un rendement de l'ordre de 40 % obtenu par une procédure en une seule étape.

Par ailleurs, chacun des isomères optiques D et L de l'ergothionéine peut être obtenu à partir de l'ergothionéine racémique (D,L) par analogie avec les techniques connues de résolution d'aminoacides.

D'autres buts, caractéristiques et avantages de l'invention apparaîtront clairement à la lumière de la description explicative qui va suivre faite en référence à divers exemples de réalisation de l'invention donnés simplement à titre d'illustration et qui ne sauraient en aucune façon limiter la portée de l'invention. Dans les exemples, tous les pourcentages sont donnés en poids sauf indication contraire.

Dans les exemples, toutes les réactions ont été effectuées sous atmosphère inerte d'azote, sauf exception notifiée.

Les spectres de masse ont été enregistrés sur un appareil Nermag R10-10B. Le mode d'ionisation utilisé est soit l'impact électronique (EI) à 70 electrons-volts, soit l'ionisation chimique (IC) dans l'ammoniac, soit le bombardement par atomes rapides (FAB) sur une matrice glycérol.

Les spectres ¹H et ¹³C RMN ont été enregistrés sur un appareil Varian Gemini-200. Les déplacements chimiques sont exprimés en ppm par rapport au tétraméthylsilane. Les multiplicités sont exprimées comme suit: "s" pour singulet, "sl" pour singulet large, "d" pour doublet, "t" pour triplet, "q" pour quadruplet et "m" pour multiplet.

Les points de fusion (pF^{•}C) ont été enregistrés sur un appareil Gallenkamp et sont donnés non corrigés.

Le pouvoir rotatoire (α_{D}) a été mesuré sur un appareil Perkin Elmer 241 à 25°C sur la raie D du sodium.

Les purifications par chromatographie liquide sur colonne ont été effectuées, selon les cas, avec de la silice Merck^{**R**} Si60 F₂₅₄ ou avec de la cellulose microcristalline Merck^{**R**}.

### Exemple 1 : Préparation de la D.L-ergothionéine :

### A- Préparation du dichlorhydrate de l'ester méthylique de L-(+)-N_{α} ,N_{α}-diméthyl-histidine :

A une solution du dichlorhydrate de l'ester méthylique de la L-(+)-histidine (Janssen; 18,16 g ; 75 mmoles) dans 150 ml d'eau déionisée est ajoutée une solution aqueuse de formaldéhyde 37 % (Janssen ; 12,29 g ; 150 mmoles). Le mélange est hydrogéné sous pression (7b) en présence de Palladium sur charbon 10 % (Aldrich; 1,0 g) pendant 5 h à température ambiante. Le catalyseur est filtré puis rincé à l'eau ; le filtrat est évaporé à sec sous vide pour donner le produit attendu sous forme d'une huile (20,3 g) qui est utilisée directement dans l'étape suivante.

### Caractéristiques physiques :

* ¹H RMN (200Mhz, D₂O) :
2,91 ppm (s ; 6H) ; 3,50 ppm (m ; 2H) ; 3,72 ppm (s ; 3H) ; 4,48 ppm (dd ; J=5,54-9,04Hz ; 1H) ; 7,38 ppm (s ; 1H); 8,61 ppm (s ; 1H).

### B - Préparation de l'ester méthylique de la (D,L)-2'-mercapto-N_{α} ,N_{α}-diméthyl-histidine :

Le dichlorhydrate de l'ester méthylique de la N_{α},N_{α} -diméthyl-histidine (27,0 g ; 100 mmoles) est dissous dans 450 ml d'eau. On y ajoute lentement du bicarbonate de sodium (Labosi ; 58,8 g ; 700 mmoles) puis 450 ml de THF. A ce mélange sous agitation, on additionne goutte à goutte pendant 30 mn du chlorothionoformiate de phényle (Lancaster ; 34,5 ml ; 250 mmoles). Le mélange réactionnel est agité pendant 24 h à 80°C. Le mélange biphasique est ensuite décanté. Les deux phases sont séparées et la phase organique est séchée. Le solvant est évaporé sous vide. Le résidu de la phase organique est chromatographié sur SiO₂ (éluant: AcOEt) pour donner 12,02g d'ester attendu. Rendement de l'ester méthylique de la 2'-mercapto-N_{α},N_{α}-diméthylhistidine: 52 %.

### Caractéristiques physiques :

* pF : 171-173°C
* ¹H RMN (200Mhz, CDCl₃) :
   2,39 ppm (s ; 6H) ; 2,78 ppm (d ; J=7,30Hz ; 2H) ; 3,38 ppm (t ; J=7,30Hz ; 1H); 3,72 ppm (s ; 3H) ; 6,44 ppm (s ; 1H); 10,08 ppm (s1 ; 1H); 10,24 ppm (s1 ; 1H).
* ¹³C RMN (50Mhz, DMSO-d₆) :
   24,52 ppm (t) ; 41,03 ppm (q ) ; 51,03 ppm (d) ; 65,04 ppm (q); 112,74 ppm (d) ; 126,13 ppm (s) ; 160,35 ppm (s) ; 171,17 ppm (s).
* SM(EI, 70eV):
   229 (M+, 25) ; 170 (8) ; 116(100).

### C - Préparation de l'ester méthylique de la (D,L)-1'-éthoxycarbonyl-2'-éthoxycarbonylthio-N_{α},N_{α}-diméthylhistidine:

L'ester méthylique de la 2'-mercapto-Nα,Nα-diméthylhistidine (12,80 g ; 55,9 mmoles) est dissous dans 200ml de dichlorométhane sous atmosphère inerte. La triéthylamine (Janssen ; 23,3 ml ; 168 mmoles) est additionnée. Puis on ajoute goutte à goutte, à 0°C, le chloroformiate d'éthyle (Janssen ; 12,8 ml ; 134mmoles) pendant 30 mn. Le mélange réactionnel est agité pendant 1 h à température ambiante, puis hydrolysé par addition de 150 ml d'une solution aqueuse saturée de NaCl. La phase organique est décantée, puis lavée avec le même volume de solution aqueuse saturée en NaCl, séchée sur MgSO₄ et évaporée à sec sous vide. Le produit brut est purifié par chromatographie sur colonne de silice (éluant : AcOEt/Cyclohexane 1/1) pour conduire à 18,71 g de produit désiré ( rendement : 90 %).

### Caractéristiques physiques :

* ¹H RMN (200Mhz, CDCl₃) :
   1,25 ppm (t, J=7,16Hz ; 3H) ; 1,35 ppm (t, J=7,16Hz ; 3H) ; 2,31 ppm (s, 6H); 2,81 ppm (dd, J=6,54-14,66Hz ; 1H) ; 2,96 ppm (dd, J=8,46-14,66Hz ; 1H); 3,60 ppm (dd, J=6,54-8,46Hz ; 1H) ; 3,62 ppm (s, 3H) ; 4,26 ppm (q, J=7,16Hz ; 2H) ; 4,36 ppm (q, J=7,16Hz ; 2H) ; 7,41 ppm (s, 1H).
* 13C RMN (50Mhz, CDCl₃) :
   172,41 ppm (s) ; 167,40 ppm (s) ; 148,55 ppm (s) ; 140,76 ppm (s) ; 135,12 ppm (s) ; 120,15 ppm (d) ; 66,89 ppm (q) ; 65,17 ppm (t) ; 64,88 ppm (t) ; 51,51 ppm (d) ; 41,99 ppm (q); 27,97 ppm (t) ; 14,37 ppm (q); 14,18 ppm (q).
* SM (EI, 70Ev) :
   373 (M+, 6) ; 314 (26) ; 116 (100).

### D - Préparation de l'ester méthylique de la (D,L)-1'-éthoxycarbonyl-2'-éthoxycarbonylthio-N_{α}N_{α}N_{α}-triméthylhistidine :

A une solution d'ester méthylique de la (D,L)-1'-éthoxycarbonyl-2'-éthoxycarbonylthio-N_{α},N_{α}-diméthylhistidine (0,64 g ; 1,72 mmoles) dans 20 ml de méthanol est ajouté du iodométhane (Lancaster ; 0,6 ml ; 9,6 mmoles). Le mélange est agité à température ambiante sous azote pendant 70 h. Le solvant et l'iodométhane en excès sont évaporés à température ambiante sous vide pour conduire à une huile qui contient essentiellement l'ammonium attendu, et est utilisée telle quelle pour l'étape suivante.

### Caractéristiques physiques :

* ¹H RMN (200Mhz, acétone-d₆) :
   1,26 ppm (t ; J=7,16 Hz ; 3H) ; 1,37 ppm (t; J=7,16 Hz ; 3H) ; 3,50-3,60 ppm (m ; 2H) ; 3,64 ppm (s ; 9H) ; 3,74 ppm (s ; 3H) ; 4,33 ppm (q ; J=7,16Hz ; 2H) ; 4,44 ppm (q ; J=7,16 Hz ; 2H) ; 4,84 ppm (dd ; J=4,08-10,84 Hz ; 1H) ; 7,99 ppm (s ; 1H).

### E - Préparation de la D,L-ergothionéine :

L'ester méthylique de la 1'-éthoxycarbonyl-2'-éthoxycarbonylthio-N_{α},N_{α},N_{α}-triméthylhistidine (0,85 g ; 1,65 mmoles) est dissous dans 25 ml de triéthylamine/eau/méthanol (1:4:6). La solution est chauffée à 60°C pendant 45 h. Les solvants sont évaporés à sec sous vide pour conduire à un résidu solide. Celui-ci est purifié par chromatographie sur une colone de cellulose (éluant: MeOH/H₂O 9/1) fournissant la D,L-ergothioneine (0,35g, 89 %).

### Caractéristiques physiques :

* pF : 275°C (déc.) ; recristallisé dans EtOH/H₂O=1:1.
* ¹H RMN (200Mhz, D₂O)
   3,01-3,18 ppm (s, 9H superposé à m, 2H) ; 3,80 ppm (dd, J=4,52-11,24Hz, 1H); 6,70 ppm (s, 1H).
* ¹³C RMN (50Mhz, D₂O) :
   173,11 ppm (s), 158,82 ppm (s), 126,68 ppm (s), 118,11 ppm (d), 79,82 ppm (d), 54,79 ppm (q), 25,38 ppm (t).

Ces spectres de RMN sont identiques à ceux obtenus à partir d'un échantillon commercial d'ergothionéine (ICN, France).
* UV (H₂O, c = 36,7 µM) : λmax = 257 nm. Cette valeur est en concordance avec les indications données dans le Merck Index, 11e éd. (1989).

Le rendement global de préparation de la D,L-ergothionéine obtenue par cette procédure est de 41%.

### Exemple 2 : Préparation de la L-(+)-ergothionéine :

### A - Préparation de l'ester méthylique de la L-(+)-2'-mercapto-N_{α},N_{α} -diméthyl-histidine :

Le dichlorhydrate de l'ester méthylique de la L-(+)-N_{α},N_{α} -diméthylhistidine (60,0 g ; 222 mmoles) est dissous dans 750 ml d'eau déionisée. Du bicarbonate de sodium solide ( Labosi ; 130,5 g ; 1,55 moles) est ajouté lentement, suivi de 750 ml de THF. Sous agitation vigoureuse et à une température comprise entre 5-10°C, le chlorothionoformiate de phényle (Lancaster ; 76ml ; 555 mmoles) est additionné pendant 30 min. Le mélange réactionnel est agité, à température ambiante, pendant 260 h. La phase aqueuse est décantée puis extraite avec du chlorure de méthylène (3X150 ml). Les phases organiques sont rassemblées puis évaporées à sec sous vide, à température ambiante. Le résidu est, ensuite, purifié par chromatographie sur colonne de silice en utilisant un gradient d'élution (AcOEt-AcOEt/MeOH=100 %-95/5) pour donner 24,0 g d'un produit pur. Ce produit (5,0 g) est suspendu dans 150 ml de chlorure de méthylène puis filtré. Le résidu obtenu après évaporation du solvant du filtrat conduit à 4,42 g d'un produit énantiomériquement pur (Rendement = 42 %).

La pureté énantiomérique est déterminée par ¹H RMN dans CDCl₃ avec 3 mg d'échantillon et 10 mg de Eu(tfc)₃.

### Caractéristiques physiques :

* pF : 170-171°C
* ¹H RMN (200MHz, CDCl₃) :
   2,39 ppm (s ; 6H) ; 2,78 ppm (d ; J=7,3Hz ; 2H) ; 3,38 ppm (t ; J=7,30Hz ; 1H); 3,72 ppm (s ; 3H) ; 6,44 ppm (s ; 1H); 10,08 ppm (sl ; 1H); 10,24 (sl ; 1H).
* ¹³C RMN (50MHz, DMSO) : 24,52 ppm (t) ; 41,03 ppm (q) ; 51,03 ppm (d) ; 65,04 ppm (q) ; 112,74 ppm (d) ; 126,13 ppm (s) ; 160,35 ppm (s) ; 171,17 ppm (s).
* SM (EI, 70Ev) :
   229 (M+, 25), 170 (8), 116 (100).
* α_{D} (c = 1,0 ; MeOH) = +31,2°

### B - Préparation de l'ester méthylique de la L-(+)-1'-éthoxycarbonyl-2'-éthoxycarbonylthio-N_{α},N_{α}-diméthylhistidine:

A une solution du composé précédent (3,85 g ; 16,8 mmoles) dans 80 ml de chlorure de méthylène, refroidie à 10°C, est ajoutée la triéthylamine (Janssen ; 5,85 ml ; 42,0 mmoles). Le chloroformiate d'éthyle (Janssen; 3,5 ml ; 37,0 mmoles) est additionné goutte à goutte, à cette température. A la fin d'addition, un précipité de chlorhydrate de triéthylamine apparait. Le mélange réactionnel est agité 0,5 h à 10°C, puis l'excès de chloroformiate d'éthyle est hydrolysé par l'addition de 50 ml d'eau. La phase organique est décantée, lavée à l'eau (2X50 ml) et finalement séchée sur MgSO₄. L'évaporation du solvant sous pression réduite conduit à 6,19 g d'une huile très légèrement jaune qui est utilisée telle quelle dans l'étape suivante (rendement : 99 %).

### Caractéristiques physiques:

* ¹H RMN (200Mhz, CDCl₃) :
   1,28 ppm (t, J=7,16Hz ; 3H) ; 1,40 ppm (t, J=7,16Hz ; 3H) ; 2,33 ppm (s, 6H); 2,83 ppm (dd, J=6,50-14,60Hz ; 1H) ; 2,98 ppm (dd, J=8,50-14,60Hz ;1H) ; 3,65 ppm (dd, J=6,50-8,50Hz ; 1H); 3,68 ppm (s, 3H) ; 4,28 ppm (q ; J=7,16Hz ; 2H) ; 4,42 ppm (q, J=7,16Hz ; 2H) ; 7,44 ppm (s, 1H).
* SM (EI, 70Ev) :
   373 (M+, 9) ; 314 (29) ; 116 (100).
* α_{D} (c=1,1 ; CH₂Cl₂) = -5,8^{•}

### C - Préparation de l'ester méthylique de la L-(+1)-1'-éthoxycarbonyl-2'-éthoxy-carbonylthio-N_{α},N_{α},N_{α}-triméthylhistidine:

Le produit de l'étape précédente (6,19 g ; 16,8 mmoles) est dissous dans 60 ml de THF anhydre; l'iodométhane (Aldrich ; 2,0 ml ; 32,1 mmoles) est ajouté goutte à goutte. Le mélange réactionnel est agité pendant 24 h à température ambiante. Le précipité est filtré puis rincé avec du THF pour donner 7,03 g de produit attendu sous forme d'iodure. Le filtrat est concentré à 10 ml environ. Après 24 h à température ambiante, on récupère 0,33 g de produit supplémentaire également sous forme d'iodure. Le rendement global de ces deux dernières étapes est de 85 %.

### Caractéristiques physiques :

* pF : 136°C (dec.)
* ¹H RMN (200MHz,CDCl₃) :
   7,84 ppm (s ; 1H) ; 4,78 ppm (dd ; J=4,32-9,52Hz ; 1H) ; 4,36 ppm (q ; J=7,0Hz ; 2H) ; 4,25 ppm (q ; J=7,0Hz ; 2H) ; 3,73 ppm (s ; 3H) ; 3,65 ppm (s ; 9H superposé à m, 1H) ; 3,32 ppm (dd ; J=9,52-14,6Hz ; 1H); 1,38 ppm (t ; J=7,0Hz ; 3H) ; 1,26 ppm (t ; J=7,0Hz ; 3H).
* ¹³C RMN (50MHz, CDCl₃) :
   167,50 ppm (s) ; 166,96 ppm (s) ; 148,21 ppm (s) ; 136,06 ppm (s) ; 135,63 ppm (s) ; 122,26 ppm (d) ; 73,93 ppm (d) ; 65,54 ppm (t, 2 CH₂) ; 54,08 ppm (q); 53,85 ppm (q, 3 CH₃) ; 26,99 ppm (t) ; 14,40 ppm (q); 14,23 ppm (q).
* α_{D} ( c = 1,0 ; MeOH) = + 34,6^{•}

### D - Préparation de la L-(+)-Ergothionéine :

A un mélange du composé triméthylammonium précédent (2,05 g ; 4,0 mmoles) et d'acide β-mercaptopropionique (Aldrich ; 30 g ; 283 mmoles) est ajouté 100ml d'acide chlorhydrique (SDS ; 35 %). La solution homogène est portée au reflux, à l'aide un bain d'huile chauffé à 110°C, pendant 26 h. Le solvant est évaporé sous vide, le résidu est repris avec 50 ml d'eau déionisée. L'acide β-mercaptopropionique, en excès, est extrait avec de l'éther éthylique (3X50 ml). La solution aqueuse, dont le pH a été ramené à 6-7 avec une solution diluée d'ammoniaque, est à nouveau évaporée à sec sous vide. Le résidu est purifié par chromatographie sur une colonne de silice à l'aide d'un gradient d'élution (AcOEt: 100 %, puis MeOH : 100 % et finalement MeOH/H₂O : 95/5). Le produit désiré est obtenu sous forme d'un solide blanc (725 mg). Celui-ci est dissous dans 3 ml d'eau déionisée, puis à cette solution est additionné en une partie 30 ml d'éthanol absolu. Après 24 h à 4°C, le solide est filtré puis rincé avec de l'éthanol absolu (480 mg). Le filtrat est concentré, le précipité obtenu est recristallisé deux fois dans ces mêmes conditions (100 mg) (rendement : 55%).

Le rendement global de préparation de la L-(+)-ergothionéine obtenue par cette procédure est de 19,5%.

### Caractéristiques physiques :

* pF : 263°C (dec.)
* ¹H RMN (200MHz, D₂O) :
   3,17 ppm (s ; 9H superposé à m, 2H) ; 3,80 ppm (dd ; J=4,5-11,2Hz ; 1H); 6,70 ppm (s ; 1H).
* ¹³C RMN (50MHz, D₂O) :
   173,11 ppm (s) ; 158,82 ppm (s) ; 126,68 ppm (s) ; 118,11 ppm (d) ; 79,82 ppm (d) ; 54,79 ppm (q) ; 25,38 ppm (t).
* UV (H₂O, C=36,7 µmoles) : λm=257 nm.
* α_{D} (c = 1,0 ; H₂O) = +115,6^{•}
Ces valeurs sont en concordance avec les indications données dans le Merck Index, 11e éd. (1989).

### Exemple 3 : Préparation de la L-(+)-ergothionéine :

### A - Préparation de l'ester méthylique de la L-(+)-2'-mercapto-N_{α},N_{α} -diméthyl-histidine :

A une solution du dichlorhydrate de l'ester méthylique de la L-(+)-N_{α},N_{α}-diméthylhistidine (1,35 g ; 5,0 mmoles) dans 20ml d'eau déionisée sont ajoutés une solution aqueuse de bicarbonate de sodium (Labosi ; 3,36g; 40 mmoles) et 20 ml d'ether éthylique. Le chlorothionoformiate de phényle (1,80 ml ; 13,0 mmoles) est ajouté goutte à goutte à température ambiante. Le mélange réactionnel est agité 5 h à cette même température. La phase aqueuse est décantée. La phase organique est lavée à l'eau déionisée (2X50ml), séchée sur MgSO₄ et évaporée pour conduire à une huile qui est reprise par 30ml de méthanol. Cette solution traitée par de la triéthylamine (Janssen ; 2,2ml ; 15,8 mmoles) à température ambiante pendant 16 h. Le solvant est évaporé sous pression réduite. Le résidu est purifié par chromatographie sur colonne de silice (éluant: AcOEt puis AcOEt/MeOH 19/1) pour conduire au produit désiré. Celui-ci est recristallisé de la même façon que dans l'étape A de l'exemple 2 (rendement: 76 %).

Les analyses physico-chimiques sont identiques à celles du même composé obtenu dans l'étape A de l'exemple 2.

Les étapes B, C et D sont conduites exactement de la même façon que pour l'exemple 2.

Le rendement global de préparation de la L-(+)-ergothionéine obtenue par cette procédure est de 35,5 %.

## Revendications

1. Procédé de synthèse chimique des'différentes formes optiques, en particulier (D,L) et (L), de l'ergothionéine, caractérisé en ce qu'il comprend essentiellement les étapes consistant à :
a) préparer ou utiliser un sel d'ester d'histidine N_{α},N_{α} -diméthylée, si nécessaire optiquement actif ;
b) traiter ce composé, par un halogénothionoformiate d'alkyle, d'alkènyle ou d'aryle, en particulier de phényle, en présence d'une base;
c) après protection du substituant soufré du composé obtenu, transformer ce dernier en un composé de type triméthylammonium ; et
d) libérer l'ergothionéine désirée par saponification ou hydrolyse acide, selon le cas.

2. Procédé de synthèse chimique de la D,L-ergothionéine, caractérisé en ce qu'il comprend essentiellement les étapes consistant à :
a) préparer ou utiliser un sel d'ester d'histidine N_{α},N_{α} -diméthylée ;
b) traiter ce composé par un halogénothionoformiate d'alkyle, d'alkènyle ou d'aryle, en particulier de phényle, en présence d'une base;
c) protéger le substituant soufré, de préférence par acylation, encore de préférence au moyen d'un halogénoformiate d'alkyle ou de phényle, en présence d'une base, de préférence la triéthylamine ;
d) traiter le composé protégé obtenu par un agent méthylant, de préférence par un halogénure de méthyle dans un alcool ; et
e) libérer la D,L-ergothionéine par saponification, de préférence au moyen d'une amine dans un mélange eau/alcool.

3. Procédé de synthèse chimique de la L-(+)-ergothionéine, caractérisé en ce qu'il comprend essentiellement les étapes consistant à :
a) préparer ou utiliser un sel d'ester de L-histidine N_{α},N_{α}-diméthylée ;
b) traiter ce composé par un halogénothionoformiate d'alkyle, d'alkènyle ou d'aryle, en particulier de phényle, en présence d'une base;
c) protéger le substituant soufré, de préférence par acylation, encore de préférence au moyen d'un halogénoformiate d'alkyle ou de phényle, en présence d'une base, de préférence la triéthylamine ;
d) traiter le composé protégé obtenu par un agent méthylant, de préférence par un halogénure de méthyle dans un alcool ; et
e) libérer la L-(+)-ergothionéine par hydrolyse acide, de préférence au moyen d'une solution d'acide fort.

4. Procédé selon l'une des revendications 1 ou 3, caractérisé en ce que l'hydrolyse acide précitée est réalisée en présence d'un mercaptan, en particulier pour obtenir la L-(+)-ergothionéine.

5. Procédé selon la revendication 4, caractérisé en ce que le mercaptan est choisi parmi un alkyle mercaptan ou un aryle mercaptan ou l'acide β-mercaptopropionique.

6. Procédé selon l'une des revendications 1 à 5, caractérisé en ce que l'ester d'histidine précité est un ester d'alkyle en C₁-C₆, notamment un ester méthylique.

7. Procédé selon l'une des revendications 1 à 6, caractérisé en ce que le sel est un halogénure, notamment un dichlorhydrate.

8. Procédé selon l'une des revendications 1 à 7, caractérisé en ce que l'halogénothionoformiate d'aryle précité est le chlorothionoformiate de phényle.

9. Procédé selon l'une des revendications 1 à 8, caractérisé en ce que la base précitée utilisée pour le traitement de l'halogénothionoformiate précité est choisie parmi un bicarbonate, en particulier le bicarbonate de sodium, une amine ou une alkylamine, en particulier la diéthylamine ou la triéthylamine.

10. Procédé selon la revendication 9, caractérisé en ce que le traitement de l'halogénothionoformiate par la base précitée est réalisé dans un solvant polaire et de préférence en deux étapes, avantageusement dans une première étape on utilise une base faible de préférence ayant un pKa inférieur à environ 9, en particulier un bicarbonate, avantageusement en présence d'un solvant polaire favorisant la solubilité du chlorothionoformiate, par exemple de l'éther éthylique ; et dans une deuxième étape on utilise une autre base de préférence dont le pKa est supérieur à environ 10, encore mieux de type organique, avantageusement type amine ou alkylamine, en particulier diéthylamine ou triéthylamine, et avantageusement en présence d'un solvant polaire, de préférence un éther, en particulier le tétrahydrofuranne.

11. Procédé selon l'une des revendications 1, 2, 6 à 10, caractérisé en ce que la saponification précitée est réalisée au moyen d'une aminé, en particulier une alkylamine, de préférence la triéthylamine, dans un mélange eau/alcool, en particulier le méthanol.

12. Procédé selon l'une des revendications 1 à 11, caractérisé en ce que l'agent méthylant précité est un halogénure de méthyle, en particulier l'iodure de méthyle, dans un alcool, en particulier le méthanol.

## Patentansprüche

1. Verfahren zur chemischen Synthese verschiedener optischer Formen, insbesondere (D,L) und (L), von Ergothionein, dadurch gekennzeichnet, daß es im wesentlichen die Schritte umfaßt bestehend aus:
a) Herstellen oder Verwenden eines, wenn notwendig optisch aktiven, N_{α} ,N_{α}-dimethylierten Histidinestersalzes;
b) Behandeln dieser Verbindung durch ein Alkyl-, Alkenyl- oder Aryl-, insbesondere Phenylhalogenthionoformiat in Anwesenheit einer Base;
c) nach dem Schützen des Schwefel-Substituenten der erhaltenen Verbindung, Überführen dieser letzteren in eine Verbindung vom Trimethylammonium-Typ; und
d) Freisetzen des gewünschten Ergothioneins gegebenenfalls durch Verseifung oder Säurehydrolyse.

2. Verfahren zur chemischen Synthese von D,L-Ergothionein, dadurch gekennzeichnet, daß es im wesentlichen die Schritte umfaßt bestehend aus:
a) Herstellen oder Verwenden eines N_{α},N_{α}-dimethylierten Histidinestersalzes;
b) Behandeln dieser Verbindung durch ein Alkyl-, Alkenyl- oder Aryl-, insbesondere Phenylhalogenthionoformiat in Anwesenheit einer Base;
c) Schützen des Schwefel-Substituenten, vorzugsweise durch Acylierung, bevorzugter mittels eines Alkyl- oder Phenylhalogenformiats, in Anwesenheit von einer Base, vorzugsweise Triethylamin;
d) Behandeln der erhaltenen geschützten Verbindung durch ein Methylierungsmittel, vorzugsweise durch ein Methylhalogenid in einem Alkohol; und
d) Freisetzen des D,L-Ergothioneins durch Verseifung, vorzugsweise mittels eines Amins in einer Wasser/Alkohol-Mischung.

3. Verfahren zur chemischen Synthese von L-(+)-Ergothionein, dadurch gekennzeichnet, daß es im wesentlichen die Schritte umfaßt bestehend aus:
a) Herstellen oder Verwenden eines N_{α},N_{α}-dimethylierten L-Histidinestersalzes;
b) Behandeln dieser Verbindung durch ein Alkyl-, Alkenyl- oder Aryl-, insbesondere Phenylhalogenthionoformiat in Anwesenheit einer Base;
c) Schützen des Schwefel-Substituenten, vorzugsweise durch Acylierung, bevorzugter mittels eines Alkyl- oder Phenylhalogenformiats, in Anwesenheit von einer Base, vorzugsweise Triethylamin;
d) Behandeln der erhaltenen geschützten Verbindung durch ein Methylierungsmittel, vorzugsweise durch ein Methylhalogenid in einem Alkohol; und
d) Freisetzen des L-(+)-Ergothioneins durch Säurehydrolyse, vorzugsweise mittels einer starken Säurelösung.

4. Verfahren nach einem der Ansprüche 1 oder 3, dadurch gekennzeichnet, daß die genannte Säurehydrolyse in Anwesenheit eines Mercaptans durchgeführt führt, insbesondere um L-(+)-Ergothionein zu erhalten.

5. Verfahren nach Anspruch 4, dadurch gekennzeichnet, daß das Mercaptan aus einem Alkylmercaptan oder einem Arylmercaptan oder β-Mercaptopropionsäure ausgewählt wird.

6. Verfahren nach einem der Ansprüche 1 bis 5, dadurch gekennzeichnet, daß der genannte Histidinester ein C₁-C₆-Alkylester, insbesondere ein Methylester, ist.

7. Verfahren nach einem der Ansprüche 1 bis 6, dadurch gekennzeichnet, daß das Salz ein Halogenid, insbesondere ein Dichlorhydrat, ist.

8. Verfahren nach einem der Ansprüche 1 bis 7, dadurch gekennzeichnet, daß das genannte Arylhalogenthionoformiat Phenylchlorthionoformiat ist.

9. Verfahren nach einem der Ansprüche 1 bis 8, dadurch gekennzeichnet, daß die genannte Base, die zur Behandlung des genannten Halogenthionoformiats verwendet wird, aus einem Bicarbonat, insbesondere Natriumbicarbonat, einem Amin oder einem Alkylamin, insbesondere Diethylamin oder Triethylamin, ausgewählt wird.

10. Verfahren nach Anspruch 9, dadurch gekennzeichnet, daß die Behandlung des Halogenthionoformiats durch die genannte Base in einem polaren Lösungsmittel und vorzugsweise in zwei Schritten durchgeführt wird, wobei vorteilhaft in einem ersten Schritt eine schwache Base vorzugsweise mit einem pKₐ-Wert von weniger als etwa 9, insbesondere ein Bicarbonat, vorteilhaft in Anwesenheit von einem polaren Lösungsmittel, das die Löslichkeit des Chlorthionoformiats fördert, beispielsweise Ethylester, verwendet wird; und in einem zweiten Schritt eine andere Base, deren pKₐ-Wert vorzugsweise mehr als etwa 10 beträgt, bevorzugter vom organischen Typ, vorteilhaft vom Amin- oder Alkylamin-Typ, insbesondere Diethylamin oder Triethylamin, und vorteilhaft in Anwesenheit von einem polaren Lösungsmittel, vorzugsweise einem Ether, insbesondere Tetrahydrofuran, verwendet wird.

11. Verfahren nach einem der Ansprüche 1, 2, 6 bis 10, dadurch gekennzeichnet, daß die genannte Verseifung mittels eines Amins, insbesondere eines Alkylamins, vorzugsweise Triethylamin, in einer Wasser/Alkohol-Mischung, insbesondere Methanol, durchgeführt wird.

12. Verfahren nach einem der Ansprüche 1 bis 11, dadurch gekennzeichnet, daß das genannte Methylierungsmittel ein Methylhalogenid, insbesondere Methyliodid, in einem Alkohol, insbesondere Methanol, ist.

## Claims

1. A method of chemical synthesis of the various optical forms, particularly (D,L) and (L) forms, of ergothioneine, characterised in that it essentially comprises the steps consisting in :
a) preparing or using an N_{α},N_{α}-dimethylated histidine ester salt, optically active if necessary ;
b) treating this compound, with an alkyl, alkenyl or aryl halothionoformate, particularly a phenyl halothionoformate, in the presence of a base;
c) after protecting the sulphur substituent of the compound obtained, converting the latter compound into a trimethylammonium-type compound; and
d) releasing the desired ergothioneine by saponification or by acidic hydrolysis, according to the case.

2. A method of chemical synthesis of D,L-ergothioneine, characterised in that it essentially comprises the steps consisting in :
a) preparing or using an N_{α},N_{α}-dimethylated histidine ester salt;
b) treating this compound with an alkyl, alkenyl or aryl halothionoformate, particularly a phenyl halothionoformate, in the presence of a base ;
c) protecting the sulphur substituent, preferably by acylation, more preferably by means of an alkyl or phenyl haloformate, in the presence of a base, preferably triethylamine;
d) treating the resulting protected compound with a methylating agent, preferably with a methyl halide in an alcohol ; and
e) releasing the D,L-ergothioneine by saponification, preferably by means of an amine in a water/alcohol mixture.

3. A method of chemical synthesis of L-(+)-ergothioneine, characterised in that it essentially comprises the steps consisting in :
a) preparing or using an N_{α},N_{α}-dimethylated L-histidine ester salt;
b) treating this compound with an alkyl, alkenyl or aryl halothionoformate, particularly a phenyl halothionoformate, in the presence of a base ;
c) protecting the sulphur substituent, preferably by acylation, more preferably by means of an alkyl or phenyl haloformate, in the presence of a base, preferably triethylamine ;
d) treating the resulting protected compound with a methylating agent, preferably with a methyl halide in an alcohol; and
e) releasing the L-(+)-ergothioneine by acidic hydrolysis, preferably by means of a solution of a strong acid.

4. The method according to one of claims 1 or 3, characterised in that the above-mentioned acidic hydrolysis is carried out in the presence of a mercaptan, particularly for obtaining L-(+)-ergothioneine.

5. The method according to claim 4, characterised in that the mercaptan is selected from an alkyl mercaptan or an aryl mercaptan, or β-mercaptopropionic acid.

6. The method according to one of claims 1 to 5, characterised in that the above-mentioned histidine ester is a C₁-C₆ alkyl ester, notably a methyl ester.

7. The method according to one of claims 1 to 6, characterised in that the salt is a halide, notably a dihydrochloride.

8. The method according to one of claims 1 to 7, characterised in that the above-mentioned aryl halothionoformate is phenyl chlorothionoformate.

9. The method according to one of claims 1 to 8, characterised in that the above-mentioned base used for treating the above-mentioned halothionoformate is selected from a bicarbonate, particularly sodium bicarbonate, an amine or an alkylamine, particularly diethylamine or triethylamine.

10. The method according to claim 9, characterised in that the treatment of the halothionoformate with the above-mentioned base is carried out in a polar solvent and preferably in two steps, advantageously in a first step a weak base is used preferably having a pKa of less than about 9, particularly a bicarbonate, advantageously in the presence of a polar solvent which favours the solubility of the chlorothionoformate, for example ethyl ether ; and in a second step another base is used, preferably a base the pKa of which is greater than about 10, even better an organic-type base, advantageously an amine- or alkylamine-type base, particularly diethylamine or triethylamine, and advantageously in the presence of a polar solvent, preferably an ether, particularly tetrahydrofuran.

11. The method according to one of claims 1, 2, 6 to 10, characterised in that the above-mentioned saponification is carried out by means of an amine, particularly an alkylamine, preferably triethylamine, in a water/alcohol mixture, particularly methanol.

12. The method according to one of claims 1 to 11, characterised in that above-mentioned methylating agent is a methyl halide, particularly methyl iodide, in an alcohol, particularly methanol.
